# EUROPÄISCHE PATENTSCHRIFT

(11) **EP 2 872 469 B1**
(45) Veröffentlichungstag und Bekanntmachung des Hinweises auf die Patenterteilung: **04.04.2018**
(21) Anmeldenummer: 13734436.2
(22) Anmeldetag: 09.07.2013
(51) Int. Cl.: B01D 17/04, C07C 7/10, B01D 17/02, C07C 5/29

(54) **PHASENTRENNVERFAHREN DURCH KIPPEN DER DISPERGIERRICHTUNG**
PHASE SEPARATION METHOD BY INVERTING THE DIRECTION OF DISPERSION
PROCÉDÉ DE SÉPARATION DE PHASES PAR BASCULEMENT DE LA DIRECTION DE DISPERSION

(30) Priorität: 11.07.2012 EP 12175829
(43) Veröffentlichungstag der Anmeldung: 20.05.2015
(73) Patentinhaber: BASF SE, 67056 Ludwigshafen am Rhein (DE)
(72) Erfinder: PFEIFFER, Daniel, 67435 Neustadt (DE); BITTERLICH, Stefan, 67246 Dirmstein (DE)
(86) Internationale Anmeldenummer: PCT/EP2013/064428
(87) Internationale Veröffentlichungsnummer: WO 2014/009332

(56) Entgegenhaltungen:
- WO-A1-2011/069929
- WO-A2-2010/062922
- WO-A2-2011/015661
- US-A1- 2011 155 632

## Beschreibung

Die vorliegende Erfindung betrifft ein Verfahren zur Abtrennung einer Phase (A) von einer Phase (B), wobei die Phase (A) eine höhere Viskosität als die Phase (B) aufweist, indem durch Rückleitung eines die Phase (B) im Überschuss aufweisenden Stroms die Dispergierrichtung von Phase (B) in Phase (A) nach Phase (A) in Phase (B) gekippt wird.

Das Auftrennen bzw. Abtrennen von zwei- oder mehrphasigen Gemischen, insbesondere wenn diese Gemische in Form von Dispersionen vorliegen, ist ein generelles Problem in chemischen Verfahren. Eine häufig auftretende Fallkonstellation in diesem Zusammenhang ist die Abtrennung einer ionischen Flüssigkeit (Phase mit einer höheren Viskosität) von Kohlenwasserstoffen (Phase mit einer niedrigen Viskosität).

Ionische Flüssigkeiten eignen sich unter anderem als Katalysatoren für die Isomerisierung von Kohlenwasserstoffen. Eine entsprechende Verwendung einer ionischen Flüssigkeit ist beispielsweise in WO 2011/069929 offenbart, wo eine spezielle Auswahl von ionischen Flüssigkeiten in Gegenwart eines Olefins zur Isomerisierung von gesättigten Kohlenwasserstoffen eingesetzt wird, insbesondere zur Isomerisierung von Methylcyclopentan (MCP) zu Cyclohexan.

Im Allgemeinen sind ionische Flüssigkeiten einerseits und Kohlenwasserstoffe (bzw. organische Phasen generell) andererseits nicht oder nur sehr schwer mischbar, sie bilden zwei getrennte Phasen aus. Um die genannte Katalysewirkung nutzen zu können, muss ein intensiver Kontakt zwischen organischer Phase und der ionischer Flüssigkeit hergestellt werden. Hierzu werden die beiden Phasen häufig in Rührkesseln und intensivem Rühren unter Erhalt von Dispersionen durchmischt. In Abhängigkeit von Parametern wie Art der ionischen Flüssigkeit bzw. der organischen Phase oder dem Phasenverhältnis kann die Dispersion entweder als Dispersion einer ionischen Flüssigkeit in der organischen Phase vorliegen oder es kann sich um eine Dispersion der organischen Phase in der ionischen Flüssigkeit handeln. In diesem Zusammenhang ist es häufig wünschenswert, die Phasen nach einer Reaktion/Katalyse wieder zu trennen.

Zur Auftrennung von zwei- oder mehrphasigen Gemischen, insbesondere von Dispersionen, ist die Verwendung von Koaleszierfiltern seit längerem bekannt. Beispielsweise wird in der internationalen Anmeldung PCT/IB2012/050417 (angemeldet am 30.01.2012) ein Verfahren zur Reduzierung des Wassergehaltes in Pyrolysebenzin unter Verwendung eines Koaleszierfilters, der aus Metall und/oder Glasfaser hergestellt ist, offenbart. Ein Koaleszierfilter kann jedoch nicht nur zur Wasserabtrennung aus Gemischen (Dispersionen) mit einer organischen Phase (Pyrolysebenzin) verwendet werden, sondern auch zur Abtrennung von ionischen Flüssigkeiten aus Dispersionen mit einer organischen Phase.

WO 2010/062922 offenbart ein mehrstufiges Verfahren zur Trennung einer ionischen Flüssigkeit von Kohlenwasserstoffen unter Verwendung eines Koaleszierfilters. Das Koaleszierfiltermaterial muss so beschaffen sein, dass es eine stärkere Affinität für die ionische Flüssigkeit gegenüber den Kohlenwasserstoffen hat. Als Koaleszierfiltermaterialien eignen sich gemäß WO 2010/062922 Glaskugeln, Edelstahl, Glasfasern, Polymerfasern oder organische Membrane, insbesondere Glasfasern. Im Koaleszierfilter wird eine Trennung der ionischen Flüssigkeit aus den Kohlenwasserstoffen bewirkt.

US-A 2011/0155632 offenbart ein Verfahren zur Herstellung von Produkten mit einem niedrigen Halogenwasserstoff-Gehalt, wobei in zumindest zwei Trennstufen durch Strippen oder Destillation aus einem Gemisch, das aus einem Reaktor stammt und eine ionische Flüssigkeit als Katalysator enthält, der Gehalt an Halogenwasserstoffen reduziert wird. In einer Ausführungsform des in US-A 2011/0155632 beschriebenen Verfahrens wird in einen Alkylierungsreaktor aus einem nachgeschalteten Phasenscheider die als Katalysator eingesetzte ionische Flüssigkeit rückgeleitet, aus einer zum Phasenscheider stromabwärts befindlichen ersten Destillationskolonne wird Chlorwasserstoff und aus einer weiter stromabwärts befindlichen zweiten Destillationskolonne wird ein Isobutan enthaltender Strom in den Alkylierungsreaktor rückgeleitet. In US-A 2011/0155632 es jedoch nirgendwo offenbart, ob die sich im jeweiligen Reaktor, in dem beispielsweise eine Alkylierung oder Isomerisierung durchgeführt wird, befindlichen oder die daraus abgezogenen Reaktionsgemische als Dispersionen vorliegen bzw. welche Dispergierrichtung gegebenenfalls vorliegen würde. Weiterhin ist in diesem Dokument auch kein Anhaltspunkt zu finden, dass durch die Rückleitung einer Phasenkomponente die Dispergierrichtung einer solchen Dispersion gekippt werden kann. Eine sinngemäße Offenbarung wie in US-A 2011/0155632 ist in US-A 2011/0155640 enthalten, allerdings betrifft das dort beschriebene Verfahren eine Kohlenwasserstoffkonversion.

Die der vorliegenden Erfindung zugrunde liegende Aufgabe besteht in der Bereitstellung eines neuen Verfahrens zur Abtrennung einer Phase mit einer höheren Viskosität, insbesondere einer ionischen Flüssigkeit, von einer Phase mit einer niedrigeren Viskosität, insbesondere einer kohlenwasserstoffhaltigen organischen Phase, wobei die Phase mit höherer Viskosität in der Phase mit niedrigerer Viskosität dispergiert ist.

Gelöst wird die Aufgabe durch ein Verfahren zur Abtrennung einer Phase (A) von einer Phase (B), wobei die Phase (A) eine höhere Viskosität als die Phase (B) aufweist, umfassend die folgenden Schritte:
a) Bereitstellung eines Stroms (S1) enthaltend eine Dispersion (D1), in der die Phase (B) in der Phase (A) dispergiert ist, und die Phase (B) zu maximal 25 Gew.-% in der Dispersion enthalten ist,
b) Einleiten eines Stroms (S2), enthaltend zu mindestens 70 Gew.-%, bevorzugt mindestens 90 Gew.-%, an Phase (B), in den Strom (S1), wobei der Strom (S2) aus Schritt f) rückgeführt wird,
c) unter Ausbildung eines Stroms (S3), enthaltend eine Dispersion (D2), in der die Phase (A) in der Phase (B) dispergiert ist, wobei das Phasenverhältnis der Phase (A) zur Phase (B) in der Dispersion (D2) ≤ 3 [kg/kg] ist,
d) Einleiten des Stroms (S3) in eine Phasentrenneinheit,
e) Auftrennen des Stroms (S3) in der Phasentrenneinheit in einen Strom (S5), enthaltend mindestens 70 Gew.-%, bevorzugt mindestens 90 Gew.-%, an Phase (B), und in einen Strom (S4) enthaltend mindestens 70 Gew.-%, bevorzugt mindestens 90 Gew.-%, an Phase (A),
f) Abtrennung einer Teilmenge des Stroms (S5) als Strom (S2) und Rückführung des Stroms (S2) nach Schritt b).

Durch das erfindungsgemäße Verfahren kann in vorteilhafter Weise eine effektive Abtrennung einer höherviskosen Phase, insbesondere von ionischen Flüssigkeiten, von einer niederviskosen Phase, insbesondere einer kohlenwasserstoffhaltigen organischen Phase, erzielt werden, indem ein Kippen der Dispergierrichtung in der die beiden Phasen enthaltenden Dispersion durchgeführt wird. Erfindungsgemäß wird durch das Kippen der Dispergierrichtung bewirkt, dass die Dispergierrichtung von "niederviskoser Phase in höherviskoser Phase" zu "höherviskoser Phase in niederviskoser Phase" geändert wird. Der Begriff "Kippen der Dispergierrichtung" wird im nachfolgenden Text im Zusammenhang mit Schritt c) des erfindungsgemäßen Verfahrens näher definiert.

Alkylierungen oder Isomerisierungen von Kohlenwasserstoffen (organischen Phasen) können in Gegenwart von ionischen Flüssigkeiten durchgeführt werden, wobei die ionischen Flüssigkeiten in aller Regel eine höhere Viskosität aufweisen als die organischen Phasen. Diese Reaktionen laufen in aller Regel mit einer Dispergierrichtung von "niederviskoser Phase in höherviskoser Phase" ab. Die Abtrennung (Phasentrennung) größerer Mengen an solchen höherviskosen Komponenten läuft hingegen relativ langsam ab, daher ist ein im Vergleich zum erfindungsgemäßen Verfahren größerer apparativer Aufwand erforderlich. Infolge des erfindungsgemäßen Kippens der Dispergierrichtung läuft die Phasentrennung viel schneller ab, dadurch ist ein geringerer apparativer Aufwand erforderlich. Somit kann mit dem erfindungsgemäßen Verfahren in Fällen, in denen die Phasentrennung einem in zweiphasigem Gemisch durchgeführten Reaktionsschritt (beispielsweise einer Isomerisierung) nachgeschaltet ist (selbst dann, wenn dort aus reaktionstechnischen Gründen ein hohes Phasenverhältnis höherviskose Phase/niederviskose Phase eingestellt wird), eine schnelle Phasentrennung erreicht und somit der apparative Aufwand für diesen Schritt gering gehalten werden. Dies ist zum Beispiel gegeben bei Kohlenwasserstoff-Konversionen in Gegenwart katalytisch wirkender ionischer Flüssigkeiten, insbesondere Isomerisierungen, wo die ionische Flüssigkeit oder eine Mischung mehrerer ionischer Flüssigkeiten die höherviskose Phase darstellt.

Sofern im Rahmen der vorliegenden Erfindung im Anschluss an das Kippen der Dispergierrichtung und des damit verbundenen Abtrennungsschritts in einer Phasentrenneinheit, vorzugsweise in einem Phasenscheider, ein weiterer Trennschritt unter Verwendung eines Koaleszierfilters, z.B. eines Koaleszierfilters aus Acrylphenolharz, oder unter Verwendung eines Nachabscheiders durchgeführt wird, wird zusätzlich eine verbesserte Abtrennung von in feindisperser Form und/oder in kleinen Mengen vorliegenden höherviskosen Komponenten, insbesondere ionische Flüssigkeiten, erreicht.

Nachfolgend wird das erfindungsgemäße Verfahren zur Abtrennung einer Phase (A) von einer Phase (B) durch Rückleitung eines im Überschuss die Phase (B) aufweisenden Stroms und dem damit verbundenen Kippen der Dispergierrichtung näher definiert.

Im Rahmen der vorliegenden Erfindung ist die Phase (A) zunächst dadurch charakterisiert, dass sie eine höhere Viskosität als die Phase (B) aufweist. In der Phase (A) können somit beliebige Verbindungen enthalten sein, unter der Voraussetzung, dass die genannte Zweiphasigkeit gegeben ist und die Viskosität der Phase (A) infolge der darin enthaltenen Verbindungen höher (größer) ist als die Viskosität der Phase (B), die wiederum durch die in der Phase (B) enthaltenen Verbindungen festgelegt wird. Beispielsweise sind in der Phase (A) ionische Flüssigkeiten enthalten, die die Viskosität der Phase (A) festlegen beziehungsweise - sofern weitere mit ionischen Flüssigkeiten mischbare Verbindungen in der Phase (A) enthalten sind- die Viskosität der Phase (A) maßgeblich beeinflussen.

Vorzugsweise weist die Phase (A) eine um mindestens 0,1 mPas, insbesondere um mindestens 20 mPas, höhere Viskosität auf als die Phase (B).

Die Phase (A) enthält vorzugsweise mindestens eine ionische Flüssigkeit. Beispielsweise können in der Phase (A) Gemische aus zwei oder mehr ionischen Flüssigkeiten enthalten sein, vorzugsweise enthält die Phase (A) eine ionische Flüssigkeit. Neben der ionischen Flüssigkeit können in der Phase (A) auch weitere Komponenten enthalten sein, die mit der ionischen Flüssigkeit mischbar sind. Bei solchen Komponenten kann es sich beispielsweise um Cokatalysatoren handeln, die bei Isomerisierungsreaktionen unter Verwendung von ionischen Flüssigkeiten eingesetzt werden. Ein bevorzugtes Beispiel für solche Cokatalysatoren sind Halogenwasserstoffe, insbesondere Chlorwasserstoff. Darüber hinaus können in der Phase (A) auch Bestandteile der Phase (B) oder Zerfallsprodukte der ionischen Flüssigkeiten, die beispielsweise während des Isomerisierungsprozesses entstehen können, enthalten sein, wie Aluminiumchlorid. Vorzugsweise ist bei der Phase (A) der Anteil an ionischer Flüssigkeit größer als 80 Gew.-% (bezogen auf die Summe aller Komponenten der Phase (A)).

Als ionische Flüssigkeiten eignen sich im Rahmen der vorliegenden Erfindung prinzipiell alle dem Fachmann bekannten ionischen Flüssigkeiten. Ein Überblick hinsichtlich geeigneter ionischer Flüssigkeiten kann beispielsweise WO 2011/069929 entnommen werden. Bevorzugt ist im Rahmen der vorliegenden Erfindung eine saure ionische Flüssigkeit. Vorzugsweise ist die in der Phase (A) enthaltene ionische Flüssigkeit eine ionische Flüssigkeit, insbesondere eine saure ionische Flüssigkeit, mit der Zusammensetzung K1AlₙX₍₃ₙ₊₁₎, wobei K1 ein einwertiges Kation, X gleich Halogen und 1 < n < 2,5, ist. K1 ist vorzugsweise ein unsubstituiertes oder zumindest teilweise alkyliertes Ammoniumion oder ein heterocyclisches (einwertiges) Kation, insbesondere ein Pyridiniumion, ein Imidazoliumion, ein Pyridaziniumion, ein Pyrazoliumion, ein Imidazoliniumion, ein Thiazoliumion, ein Triazoliumion, ein Pyrrolidiniumion, ein Imidazolidiniumion oder ein Phosphoniumion. X ist vorzugsweise Chlor oder Brom.

Mehr bevorzugt enthält die ionische Flüssigkeit, insbesondere die saure ionische Flüssigkeit, als Kation ein zumindest teilweise alkyliertes Ammoniumion oder ein heterocyclisches Kation und/oder als Anion ein Chloroalumination mit der Zusammensetzung AlₙCl₍₃ₙ₊₁₎ mit 1 < n < 2,5. Vorzugsweise enthält das zumindest teilweise alkylierte Ammoniumion einen, zwei oder drei Alkylreste mit (jeweils) 1 bis 10 Kohlenstoffatomen. Sofern zwei oder drei Alkylsubstituenten mit den entsprechenden Ammoniumionen vorhanden sind, kann die jeweilige Kettenlänge unabhängig voneinander gewählt werden, vorzugsweise weisen alle Alkylsubstituenten die gleiche Kettenlänge auf. Besonders bevorzugt sind trialkylierte Ammoniumionen mit einer Kettenlänge von 1 bis 3 Kohlenstoffatomen. Das heterocyclische Kation ist vorzugsweise ein Imidazoliumion oder ein Pyridiniumion.

Besonders bevorzugt enthält die ionische Flüssigkeit, insbesondere die saure ionische Flüssigkeit, als Kation ein zumindest teilweise alkyliertes Ammoniumion und als Anion ein Chloroalumination mit der Zusammensetzung AlₙCl₍₃ₙ₊₁₎ mit 1 < n < 2,5. Beispiele für solche besonders bevorzugten ionischen Flüssigkeiten sind Trimethylammoniumchloroaluminat und Triethylammoniumchloroaluminat.

Die Phase (B) ist im Rahmen der vorliegenden Erfindung zunächst dadurch charakterisiert, dass sie gegenüber der Phase (A) eine niedrigere Viskosität aufweist. Beispielsweise kann es sich bei der Phase (B) um eine organische Phase handeln. Vorzugsweise ist die Phase (B) eine organische Phase, die nicht oder nur sehr schwer mit ionischen Flüssigkeiten mischbar ist und/oder die maximal 1 Gew.-% an ionischen Flüssigkeiten (bezogen auf das Gesamtgewicht der Phase) enthält (exklusiv evtl. eindispergierter Fremdphase). Weiterhin ist es bevorzugt, dass die Phase (B) mindestens einen Kohlenwasserstoff enthält. Mehr bevorzugt enthält die Phase (B) als Kohlenwasserstoff Cyclohexan oder ein Gemisch aus Cyclohexan mit mindestens einem weiteren Kohlenwasserstoff, ausgewählt aus Methylcyclopentan (MCP), n-Hexan, iso-Hexan, n-Heptan, iso-Heptan oder Dimethylcyclopentan. Besonders bevorzugt enthält die Phase (B) ein Gemisch aus Cyclohexan, MCP und mindestens einem weiteren Kohlenwasserstoff.

Im Rahmen der vorliegenden Erfindung erfolgt in Schritt a) die Bereitstellung eines Stroms (S1) enthaltend eine Dispersion (D1), in der die Phase (B) in der Phase (A) dispergiert ist, und die Phase (B) zu maximal 25 Gew.-% in der Dispersion enthalten ist. Die Dispergierrichtung (das heißt die Information, welche Phase in disperser Form in der der jeweils anderen Phase vorliegt) kann bestimmt werden, indem eine Probe, gegebenenfalls nach Zusatz eines selektiv die eine Phase einfärbenden Farbstoffs, unter einem Lichtmikroskop mit Durchlicht untersucht wird. Das Vorliegen einer Dispersion als solcher kann anhand der Trübung des entsprechenden Gefäßinhalts erkannt werden.

Die Dispersion (D1) kann nach dem Fachmann bekannten Methoden hergestellt werden, beispielsweise kann eine solche Dispersion durch intensives Verrühren der in den jeweiligen Phasen enthaltenen Komponenten erzeugt werden. Ein solcher Vorgang kann beispielsweise im Rahmen eines Isomerisierungsverfahrens von Kohlenwasserstoff unter Verwendung einer ionischen Flüssigkeit stattfinden. In der Dispersion (D1) können die Phasen (A) und (B) in beliebigen Verhältnissen zueinander vorliegen, unter der Voraussetzung, dass die Phase (B) in der Phase (A) dispergiert ist. Im Strom (S1) in der Dispersion (D1) ist die Phase (B) zu maximal 25 Gew.-%, enthalten (jeweils bezogen auf die Menge an Phase (A)).

Gemäß dem erfindungsgemäßen Schritt b) erfolgt das Einleiten eines Stroms (S2), enthaltend zu mindestens 70 Gew.-%, bevorzugt mindestens 90 Gew.-%, insbesondere mindestens 99 Gew.-%, an Phase (B), in den Strom (S1), wobei der Strom (S2) aus Schritt f) rückgeführt wird. Die vorstehenden Angaben in Gew.-% beziehen sich auf die Gesamtmenge des Stroms (S2).

In Schritt c) erfolgt die Ausbildung eines Stroms (S3), enthaltend eine Dispersion (D2), in der die Phase (A) in der Phase (B) dispergiert ist, wobei das Phasenverhältnis der Phase (A) zur Phase (B) in der Dispersion (D2) < 3 [kg/kg] ist. Durch die Rückleitung einer Teilmenge des Stroms (S5) als Strom (S2) und der damit verbundenen Einleitung des Stromes (S2) in den Strom (S1) wird ein Kippen der Dispergierrichtung im Strom (S1) erzielt. Kippen der Dispergierrichtung bedeutet, dass im Strom (S1) zunächst eine Dispersion (D1) enthalten ist, bei der die Phase (B) in der Phase (A) dispergiert ist. Die Menge des Stromes (S2) in Schritt b) wird so gewählt, dass ein Strom (S3) ausgebildet wird, der die Dispersion (D2) enthält, in der die Phase (A) in der Phase (B) dispergiert ist. Im Rahmen der vorliegenden Erfindung ist der Schritt c) vorzugsweise die direkte Konsequenz der Durchführung des Schritts b). Dies bedeutet, dass die erfindungsgemäßen Schritte b) und c) vorzugsweise räumlich nicht getrennt voneinander durchgeführt werden, sondern die Durchführung des Schritts b) unmittelbar die Ausbildung der Dispersion (D2) gemäß des Schritts c) bewirkt. Vorzugsweise erfolgt in Schritt b) das Einleiten des Stroms (S2) in den Strom (S1) in einen Rührbehälter oder statischen Mischer, in dem der Strom (S3) gemäß Schritt c) ausgebildet wird. Gegebenenfalls ist es aber auch möglich, dass die erfindungsgemäßen Schritte b) und c) räumlich getrennt voneinander durchgeführt werden. Bei allen Ausführungsformen ist es auch denkbar, dass in Schritt (b) ein oder mehrere weitere Ströme eingeleitet werden, die einen Überschuss an Phase (A) oder an Phase (B) enthalten, um somit die Einstellung der Dispersion (D2) zu steuern.

Gemäß Schritt d) erfolgt im erfindungsgemäßen Verfahren das Einleiten des Stroms (S3) in eine Phasentrenneinheit. Phasentrenneinheiten als solche sind dem Fachmann bekannt. Bei dieser Phasentrenneinheit handelt es sich vorzugsweise um einen Phasenscheider.

In Schritt e) erfolgt das Auftrennen des Stroms (S3) in der Phasentrenneinheit in einen Strom (S5), enthaltend mindestens 70 Gew.-%, bevorzugt mindestens 90 Gew.-%, besonders bevorzugt mindestens 99 Gew.-%, an Phase (B), und in einen Strom (S4) enthaltend mindestens 70 Gew.-% , bevorzugt mindestens 90 Gew.-%, besonders bevorzugt mindestens 99 Gew.-%, an Phase (A). Die vorstehenden Angaben in Gew.-% beziehen sich auf die entsprechenden Mengen, die im Strom (S3) enthalten sind.

In Schritt f) erfolgt die Abtrennung einer Teilmenge des Stroms (S5) als Strom (S2) und Rückführung des Stroms (S2) nach Schritt b). Beispielsweise erfolgt die Abtrennung von Strom (S2) aus Strom (S5) außerhalb der Phasentrenneinheit. In der Regel werden zwischen 50 und 90 % der Gesamtmenge des Stroms (S5) als Strom (S2) abgetrennt und in den Strom (S1) rückgeführt. Allerdings ist es auch denkbar, dass zumindest temporär größere Mengen oder der Strom (S5) sogar vollständig rückgeleitet werden. Das Phasenverhältnis der Phase (A) zur Phase (B) in der im Strom (S3) enthaltenen Dispersion (D2) ist ≤ 3 [kg/kg], besonders bevorzugt ≤ 0,9 [kg/kg] ist. In einer alternativen Ausführungsform der vorliegende Erfindung ist es bevorzugt, dass das Phasenverhältnis der Phase (A) zur Phase (B) in der im Strom (S3) enthaltenen Dispersion (D2) < 2,5 [kg/kg] ist, wobei der Strom (S3) in einer Rührvorrichtung durch Einleiten des Stroms (S2) in den Strom (S1) ausgebildet wird.

Weiterhin ist es bevorzugt, dass der Strom (S1) aus einer Reaktion in Gegenwart einer ionischen Flüssigkeit (als Katalysator) erhalten wird, vorzugsweise einer Isomerisierung, insbesondere einer Isomerisierung von Methylcyclopentan (MCP) zu Cyclohexan. Vorzugsweise wird die Reaktion in einem Rührbehälter oder einer Kaskade von Rührbehältern durchgeführt.

Weiterhin ist es bevorzugt, dass der (aus der Phasentrenneinheit) gemäß Schritt e) erhaltene Strom (S5) maximal 10 Gew.-% an Phase (A) enthält. Besonders bevorzugt ist im Strom (S5) keine oder nur eine geringe Menge an Phase (A) enthalten (< 1 Gew.-%). Weiterhin ist es bevorzugt, dass der (aus der Phasentrenneinheit) gemäß Schritt e) erhaltene Strom (S4) maximal 15 Gew.-% an Phase (B) enthält und/oder der Strom (S4) in eine Isomerisierung rückgeleitet wird. Die vorstehenden Angaben in Gew.-% beziehen sich auf die Gesamtmengen von Strom (S5) bzw. Strom (S4).

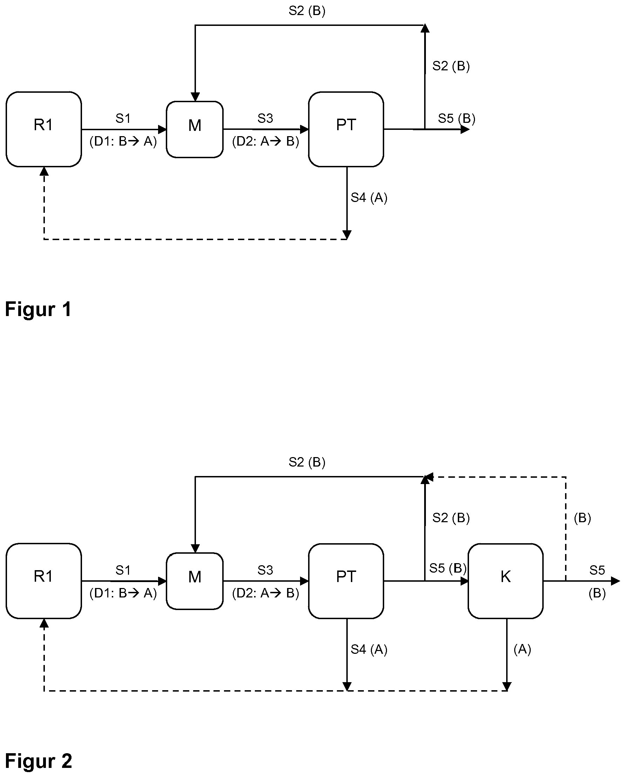

In Figur 1 wird das erfindungsgemäße Verfahren gemäß der vorstehend beschriebenen Schritte a) bis f) nochmals verdeutlicht. Zum besseren Verständnis sind in Figur 1 unter den jeweiligen Strömen in Klammern die darin enthaltenen Hauptkomponenten angegeben. Bei den Strömen (S1) und (S3) ist im jeweiligen Klammerausdruck auch die Dispergierrichtung der jeweiligen Dispersionen mit berücksichtigt, wobei der Pfeil die Dispergierrichtung zum Ausdruck bringt. Dies bedeutet, dass beispielsweise die im Strom (S1) enthaltene Dispersion (D1) eine Phase (B) aufweist, die in der Phase (A) dispergiert ist. In Figur 1 erfolgt die Einleitung des Stromes (S2) in den Strom (S1) in einer Mischvorrichtung (M). Durch die gestrichelte Linie ist angedeutet, dass der Strom (S4) gegebenenfalls auch in den Reaktionsapparat oder eine Kaskade von Reaktionsapparaten (R1) rückgeleitet werden kann, in der eine Isomerisierung in Gegenwart einer ionischen Flüssigkeit durchgeführt werden kann. PT in Figur 1 bedeutet Phasentrenneinheit.

In einer bevorzugten Ausführungsform der vorliegenden Erfindung wird mit dem Strom (S5), der in der Phasentrenneinheit gemäß Schritt e) anfällt, (mindestens) ein weiterer Phasentrennungsschritt durchgeführt. Dieser weitere Phasentrennungsschritt wird vorzugsweise durchgeführt, um im Strom (S5) enthaltene Restmengen an Phase (A) abzutrennen. Besonders bevorzugt ist im Strom (S5) nach Durchführung dieses weiteren Phasentrennungsschritt keine oder nur eine geringe Menge an Phase (A) enthalten (< 50 Gew.-ppm). Dieser weitere Phasentrennungsschritt kann direkt im Anschluss an die Durchführung des Schrittes e) erfolgen oder erst im Anschluss an die Abtrennung des Stroms (S2) gemäß Schritt f). Vorzugsweise erfolgt dieser weitere Phasentrennungsschritt im Anschluss an die Abtrennung des Stroms (S2) gemäß Schritt f).

Vorrichtungen zur Durchführung dieses weiteren Phasentrennungsschritts sind dem Fachmann bekannt. Vorzugsweise werden hierfür Vorrichtungen verwendet, die geeignet sind, Restmengen oder kleinere Mengen (< 2,5 Gew.-% bezogen auf die zu trennende Gesamtmenge) abzutrennen. Bevorzugte Vorrichtungen sind Koaleszierfilter oder andere Nachabscheider. Nachabscheider sind ein auf eine erste Phasentrenneinheit nachfolgender Phasenscheider mit oder ohne Einbauten. Mögliche Einbauten sind Gestricke, Füllkörper, Packungen oder Röhren.

Vorzugsweise wird dies so durchgeführt, dass nach Abtrennung des Stroms (S2) der Strom (S5) durch einen Koaleszierfilter und/oder einen anderen Nachabscheider geleitet wird, um im Strom (S5) verbliebene Restmengen an Phase (A) abzutrennen. Besonders bevorzugt ist im Strom (S5) nach Durchführung dieses weiteren Phasentrennungsschritts keine oder nur eine geringe Menge an Phase (A) enthalten (< 50 Gew.-ppm).

Sofern ein Koaleszierfilter verwendet wird, handelt es sich vorzugsweise um einen Koaleszierfilter aus Glasfaser oder Acrylphenolharz, insbesondere aus Acrylphenolharz. Koaleszierfilter aus Acrylphenolharzsind sind beispielsweise kommerziell erhältlich von der Firma Fuhr GmbH (Deutschland) oder von dem Hersteller CUNO Fluid Purification. Solche geeigneten Koaleszierfilter (K) haben Feinheiten von 1-150 µm, bevorzugt 10, 25 oder 50 µm, besonders bevorzugt 10 µm. Weiterhin sind 2 Versionen bezüglich der Oberfläche möglich: gerillt und ungerillt, bevorzugt ist ungerillt. Die Kerzen des Koaleszierfilter (K) als solche haben beispielsweise einen Innendurchmesser von 27 mm und einen Außendurchmesser von 65 mm und sind in Längen von 4" bis 60" erhältlich. Die Kerze ist vorzugsweise eine asymmetrische, harzgebundene und stützkernlose Filterkerze. Vorzugsweise enthält sie im Wesentlichen Acrylfasern, die mit Phenolharz gebunden werden.
Der Koaleszierfilter kann in eine größere Einheit, beispielsweise einen Filterbehälter, integriert sein. Vorzugsweise wird im Rahmen der vorliegenden Erfindung unter einem Koaleszierfilter, der aus Glasfaser oder Acrylphenolharz hergestellt ist, das Filtermaterial als solches verstanden. Die sonstigen Komponenten der Filtereinheit, beispielsweise der Behälter der Einheit (Filterbehälter) oder das Filtermodul, in dem das Filtermaterial eingebracht ist, können aus anderen Materialien als Glasfaser und/oder Acrylphenolharz hergestellt sein. Der Begriff "hergestellt aus" bedeutet im Rahmen der vorliegenden Erfindung, dass das zur Herstellung des Filtermaterials verwendete Material Glasfaser oder Acrylphenolharz enthält. Vorzugsweise enthält das Filtermaterial mindestens 50 Gew.-%, mehr bevorzugt mindestens 75 Gew.-% und insbesondere mindestens 95 Gew.-% Glasfaser oder Acrylphenolharz.

Sofern ein anderer Nachabscheider (auch als Nachphasenschneider bezeichnet) als ein Koaleszierfilter verwendet wird, enthält der Nachabscheider vorzugsweise ein Gestrick, insbesondere ein Glasfasergestrick. Geeignete Gestricke, insbesondere Glasfasergestricke, sind dem Fachmann bekannt, sie sind beispielsweise kommerziell erhältlich von der Firma Rhodius (Deutschland). Bei den bevorzugten Glasfasergestricken handelt es sich um Glasstapelfasern mit einem Faserdurchmesser zwischen 0,1 bis 0,6 mm, bevorzugt zwischen 0,14 bis 0,3 mm. Das Gestrick enthält im Wesentlichen aufgewickelten (Glasstapel-)Fasermatten mit einer Packungsdichte zwischen 100 bis 800 kg/m³, bevorzugt 150 bis 500 kg/m³, besonders bevorzugt 200 bis 400 kg/m³.

Gegebenenfalls kann die aus der Vorrichtung zur Durchführung des weiteren Phasentrennungsschritts abgetrennte Menge an Phase (A) in das erfindungsgemäße Verfahren rückgeleitet werden. Vorzugsweise erfolgt eine solche Rückleitung in den Reaktionsapparat oder die Kaskade von Reaktionsapparaten, in denen eine Isomerisierung in Gegenwart einer ionischen Flüssigkeit durchgeführt werden kann. Vorzugsweise wird diese Rückleitung mit dem Strom (S4) vereinigt, der in Schritt e) des erfindungsgemäßen Verfahrens anfällt. Gegebenenfalls können diese die Phase (A) enthaltenen Ströme auch an eine andere Stelle des erfindungsgemäßen Verfahrens rückgeleitet werden, beispielswiese in eine Misch-oder Rührvorrichtung, um die Konzentration der Phase (A) in der Dispersion (D2) zu steuern.

Außerdem ist es möglich, dass aus dem Strom (S5), nachdem in der Vorrichtung zur Durchführung des weiteren Phasentrennungsschritts die Restmenge an Phase (A) abgetrennt wurde, eine weitere Teilmenge abgetrennt wird und diese gegebenenfalls mit dem Strom (S2) vereinigt und nach Schritt b) rückgeleitet wird.

In Figur 2 wird das erfindungsgemäße Verfahren (einer Ausgestaltung) der vorstehend beschriebenen bevorzugten Ausführungsform nochmals verdeutlicht. In Figur 2 haben die Abkürzungen, Pfeile sowie gestrichene Linien eine sinngemäße Bedeutung, wie vorstehend für Figur 1 ausgeführt; K bedeutet Koalesziervorrichtung enthaltend einen Koaleszierfilter.

Aus dem Strom (S5) wird im Rahmen der vorliegenden Erfindung vorzugsweise Cyclohexan isoliert. Verfahren und Vorrichtungen zur Abtrennung von Cyclohexan aus dem Strom (S5), insbesondere wenn es sich um ein Kohlenwasserstoffgemisch handelt, sind dem Fachmann bekannt. Gegebenenfalls können vor der Abtrennung des Cyclohexans noch weitere Aufreinigungsschritte (beispielsweise eine Wäsche mit einer wässrigen und/oder alkalischen Phase) durchgeführt werden, die dem Fachmann bekannt sind.

Nachfolgend wird die Erfindung anhand der Beispiele verdeutlicht.

### Beispiele

Für den Versuch werden die folgenden Substanzen verwendet:
Phase (A):
   Ionische Flüssigkeit (IL) mit der Zusammensetzung (CH₃)₃NH AlₙCl₃ₙ₊₁ mit n=1,82 laut Elementaranalyse (auch als IL-Phase bezeichnet).
Phase (B):
   Kohlenwasserstoff-Gemisch mit der Zusammensetzung (auch als organische Phase bezeichnet)
      - Methylcyclopentan 20 Gew.-%
      - Cyclohexan 50 Gew.-%
      - Hexan 28 %
      - Isohexane (technische Mischung) 2 Gew.-%

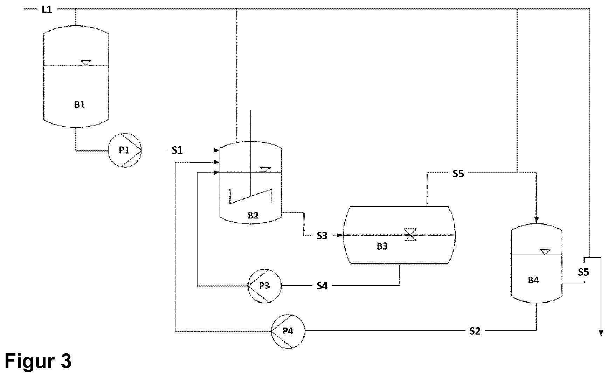

Die Versuchsanordnung ist in der Abbildung gemäß Figur 3 dargestellt:
In Behälter (B1) wird die Phase (B) vorgelegt. B1 ist (wie auch B2 und B3 und die Überläufe aus B4 und B5) mit einer Stickstoff führenden Gasleitung (L1) verbunden, die auf Atmosphärendruck gehalten wird.

Mittels der Dosierpumpe P1 wird die Phase (B) als Strom (S0) in den Rührbehälter B2 geführt. Dabei handelt es sich um einen 2L-Rührbehälter aus Glas (Innendurchmesser: 100 mm) mit einem 6-blättrigen Schrägblattrührer, Durchmesser 60 mm. B2 wird mit einem Inhalt von 1,0 Liter betrieben. In B2 wird durch Rühren mit einer Drehzahl von 1400 min⁻¹ eine Dispersion der beiden Phasen erzeugt, wobei die Phase (A) dispergiert ist, die als Strom (S1) in den mit B2 baugleichen Rührbehälter B3 geführt wird.

Die Drehzahl des Rührers in B3 wird während des Versuchs variiert, siehe Tabelle 1.

In B3 wird (S1) vereinigt mit dem Strom (S2), der aus dem Behälter B5 (Pumpvorlage) über die mengengeregelte Dosierpumpe P5 mit im Versuchsverlauf variierter Menge (siehe Tabelle 1) abgezogen wird. In B3 bildet sich eine Dispersion aus, in der die Phase (A) in der Phase (B) dispergiert ist. Aus B3 wird der Strom (S3) in den Phasenscheider B4 (Glas, liegend, Innenmaße: Durchmesser 50 mm, Länge 500 mm) geführt. In diesem Apparat, der geflutet betrieben wird, kommt es zur Trennung der IL-Phase (Unterphase) von der Kohlenwasserstoff-Phase (Oberphase). Die Unterphase wird als Strom (S4) über die Dosierpumpe P4 nach B2 mit konstanter Pumprate von 1,5 kg/h zurückgeführt.

Die Oberphase aus B4 wird als Strom (S5) über einen Überlauf, der den Flüssigkeitsstand in B2 und B3 bestimmt, in die Vorlage B5 geführt. Aus B5 wird bei einigen Versuchseinstellungen der Rückführstrom (S2) nach B2 gepumpt, der nach Abzug von Strom (S2) verbleibende Strom (S6) wird über einen Überlauf, der den Flüssigkeitsstand in B5 bestimmt, in ein Auffanggefäß geführt.

Alle Behälter sind mit einem Doppelmantel ausgestattet und werden während der nachfolgend beschriebenen Versuche mittels eines über einen Laborthermostaten zirkulierten Wärmeträgeröls auf 40 °C gehalten.

Das Phasentrennverhalten wird bei jeder Versuchseinstellung auf zwei Arten überprüft:
1. Beobachten des Phasenscheiders (Prüfen auf Vorhandensein eines Dispersionskeils und gegebenenfalls Abschätzung von dessen Länge),
2. Anhalten des Rührers in B3 nach Unterbrechung der Zuführströme (Ausschalten der Pumpen) und Messung der Zeit für den Phasenzerfall.

Die Ergebnisse sind in der folgenden Tabelle 1 dargestellt:

| Einstellung Nr. | Mengenströme [kg/h] | | | Phasenverhältnis (A)/(B) in B3* [kg/kg] | Rührerdrehzahl B3 [1/min] | Dispersions keil in B4 [cm] | Zeit für die Phasentrennung [min] |
|---|---|---|---|---|---|---|---|
| | Strom S0 | Strom S4 | Strom S2 | | | | |
| 1 (Vgl.) | 0,5 | 1,5 | 0 | 3 | 1400 | 7 | ca. 4 |
| 2 | 0,5 | 1,5 | 0,25 | 2 | 1400 | <0,1 | 0,2 |
| 3 | 0,5 | 1,5 | 0,44 | 1,6 | 1400 | <0,1 | 0,2 |
| 4 | 0,5 | 1,5 | 0,75 | 1,2 | 1200 | <0,1 | 0,2 |
| 5 | 0,5 | 1,5 | 1,17 | 0,9 | 1030 | <0,1 | 0,2 |

Im Gegensatz zum Vergleichsbeispiel 1 ist es bei den Ausführungsbeispielen 2 bis 5 zum Kippen der Dispergierrichtung gekommen. Im Phasenscheider (B4) kann dies auch am nahezu vollständigen Verschwinden der Dispersionskeils festgestellt werden. Die Länge des Dispersionskeils ist ein Maß für die Zeit, die für den Phasenzerfall benötigt wird. Die Ergebnisse zeigen weiterhin, dass es durch Rückführen von organischer Phase über Strom (S2) möglich ist, die Dispergierrichtung zum Kippen zu bringen und dadurch eine erhebliche Beschleunigung der Phasentrennung herbeizuführen.

## Patentansprüche

1. Verfahren zur Abtrennung einer Phase (A) von einer Phase (B), wobei die Phase (A) eine höhere Viskosität als die Phase (B) aufweist, umfassend die folgenden Schritte:
a) Bereitstellung eines Stroms (S1) enthaltend eine Dispersion (D1), in der die Phase (B) in der Phase (A) dispergiert ist, und die Phase (B) zu maximal 25 Gew.-% in der Dispersion enthalten ist,
b) Einleiten eines Stroms (S2), enthaltend zu mindestens 70 Gew.-%, bevorzugt mindestens 90 Gew.-%, an Phase (B), in den Strom (S1), wobei der Strom (S2) aus Schritt f) rückgeführt wird,
c) unter Ausbildung eines Stroms (S3), enthaltend eine Dispersion (D2), in der die Phase (A) in der Phase (B) dispergiert ist, wobei das Phasenverhältnis der Phase (A) zur Phase (B) in der Dispersion (D2) ≤ 3 [kg/kg] ist,
d) Einleiten des Stroms (S3) in eine Phasentrenneinheit,
e) Auftrennen des Stroms (S3) in der Phasentrenneinheit in einen Strom (S5), enthaltend mindestens 70 Gew.-%, bevorzugt mindestens 90 Gew.-%, an Phase (B), und in einen Strom (S4) enthaltend mindestens 70 Gew.%, bevorzugt mindestens 90 Gew.-%, an Phase (A),
f) Abtrennung einer Teilmenge des Stroms (S5) als Strom (S2) und Rückführung des Stroms (S2) nach Schritt b).

2. Verfahren gemäß Anspruch 1, **dadurch gekennzeichnet, dass** die Phase (A) eine um mindestens 20 mPas höhere Viskosität aufweist als die Phase (B).

3. Verfahren gemäß Anspruch 1 oder 2, **dadurch gekennzeichnet, dass** die Phase (A) mindestens eine ionische Flüssigkeit und/oder die Phase (B) mindestens einen Kohlenwasserstoff enthält.

4. Verfahren gemäß Anspruch 3, **dadurch gekennzeichnet, dass** die in der Phase (A) enthaltene ionische Flüssigkeit als Kation ein zumindest teilweise alkyliertes Ammoniumion oder ein heterocyclisches Kation und/oder als Anion ein Chloroalumination mit der Zusammensetzung AlₙCl₍₃ₙ₊₁₎ mit 1<n<2,5 enthält.

5. Verfahren gemäß Anspruch 3 oder 4, **dadurch gekennzeichnet, dass** die Phase (B) als Kohlenwasserstoff Cyclohexan oder ein Gemisch aus Cyclohexan mit mindestens einem weiteren Kohlenwasserstoff, ausgewählt aus Methylcyclopentan (MCP), n-Hexan, iso-Hexan, n-Heptan, iso-Heptan oder Dimethylcyclopentan, enthält.

6. Verfahren gemäß einem der Ansprüche 1 bis 5, **dadurch gekennzeichnet, dass** die Phasentrenneinheit ein Phasenscheider ist.

7. Verfahren gemäß einem der Ansprüche 1 bis 6, **dadurch gekennzeichnet, dass** in Schritt b) das Einleiten des Stroms (S2) in den Strom (S1) in einem Rührbehälter oder statischen Mischer erfolgt, in der der Strom (S3) gemäß Schritt c) ausgebildet wird.

8. Verfahren gemäß einem der Ansprüche 1 bis 7, **dadurch gekennzeichnet, dass** das Phasenverhältnis der Phase (A) zur Phase (B) in der im Strom (S3) enthaltenen Dispersion (D2) ≤ 0,9 [kg/kg] ist.

9. Verfahren gemäß einem der Ansprüche 1 bis 7, **dadurch gekennzeichnet, dass** das Phasenverhältnis der Phase (A) zur Phase (B) in der im Strom (S3) enthaltenen Dispersion (D2) < 2,5 [kg/kg] ist, wobei der Strom (S3) in einer Rührvorrichtung durch Einleiten des Stroms (S2) in den Strom (S1) ausgebildet wird.

10. Verfahren gemäß einem der Ansprüche 1 bis 9, **dadurch gekennzeichnet, dass** der Strom (S1) aus einer Isomerisierung erhalten wird, vorzugsweise einer Isomerisierung in Gegenwart einer ionischen Flüssigkeit, insbesondere einer Isomerisierung von Methylcyclopentan (MCP) zu Cyclohexan in Gegenwart einer ionischen Flüssigkeit.

11. Verfahren gemäß einem der Ansprüche 1 bis 10, **dadurch gekennzeichnet, dass** der gemäß Schritte) enthaltene Strom (S5) maximal 10 Gew.-% an Phase (A) enthält (bezogen auf die Menge, die in (S3) enthalten ist).

12. Verfahren gemäß einem der Ansprüche 1 bis 11, **dadurch gekennzeichnet, dass** der aus der Phasentrenneinheit gemäß Schritt e) erhaltene Strom (S4) maximal 15 Gew.-% an Phase (B) enthält (bezogen auf die Menge, die in (S3) enthalten ist) und/oder der Strom (S4) in eine Isomerisierung rückgeleitet wird.

13. Verfahren gemäß einem der Ansprüche 1 bis 12, **dadurch gekennzeichnet, dass** das Phasenverhältnis der Phase (A) zur Phase (B) in der im Strom (S1) enthaltenen Dispersion (D1) > 3 [kg/kg], bevorzugt > 4 [kg/kg] ist.

14. Verfahren gemäß einem der Ansprüche 1 bis 13, **dadurch gekennzeichnet, dass** nach Abtrennung des Stroms (S2) der Strom (S5) durch einen Koaleszierfilter und/oder einen Nachabscheider geleitet wird, um im Strom (S5) verbliebene Restmengen an Phase (A) abzutrennen.

15. Verfahren gemäß Anspruch 14, **dadurch gekennzeichnet, dass** ein Koaleszierfilter, vorzugsweise aus Glasfaser oder Acrylphenolharz, verwendet wird oder dass der Nachabscheider ein Gestrick, vorzugweise ein Glasfasergestrick, enthält.

16. Verfahren gemäß einem der Ansprüche 1 bis 15, **dadurch gekennzeichnet, dass** aus dem Strom (S5) Cyclohexan isoliert wird.

## Claims

1. A process for separating a phase (A) from a phase (B), phase (A) having a higher viscosity than phase (B), comprising the following steps:
a) providing a stream (S1) comprising a dispersion (D1) in which phase (B) is dispersed in phase (A), and phase (B) is present in the dispersion to an extent of not more than 25% by weight,
b) introducing a stream (S2) comprising at least 70% by weight, preferably at least 90% by weight, of phase (B), into stream (S1), stream (S2) being recycled from step f),
c) to form a stream (S3) comprising a dispersion (D2) in which phase (A) is dispersed in phase (B), where the phase ratio of phase (A) to phase (B) in dispersion (D2) is ≤ 3 [kg/kg],
d) introducing stream (S3) into a phase separation unit,
e) separating stream (S3) in the phase separation unit into a stream (S5) comprising at least 70% by weight, preferably at least 90% by weight, of phase (B), and into a stream (S4) comprising at least 70% by weight, preferably at least 90% by weight, of phase (A),
f) removing a portion of stream (S5) as stream (S2) and recycling stream (S2) to step b).

2. The process according to claim 1, wherein the viscosity of phase (A) is at least 20 mPas higher than that of phase (B).

3. The process according to claim 1 or 2, wherein phase (A) comprises at least one ionic liquid and/or phase (B) comprises at least one hydrocarbon.

4. The process according to claim 3, wherein the ionic liquid present in phase (A) comprises, as a cation, an at least partly alkylated ammonium ion or a heterocyclic cation and/or, as an anion, a chloroaluminate ion having the composition AlₙCl₍₃ₙ₊₁₎ where 1 < n < 2.5.

5. The process according to claim 3 or 4, wherein phase (B) comprises, as the hydrocarbon, cyclohexane or a mixture of cyclohexane with at least one further hydrocarbon selected from methylcyclopentane (MCP), n-hexane, isohexane, n-heptane, isoheptane or dimethylcyclopentane.

6. The process according to any of claims 1 to 5, wherein the phase separation unit is a phase separator.

7. The process according to any of claims 1 to 6, wherein, in step b), stream (S2) is introduced into stream (S1) in a stirred vessel or static mixer in which stream (S3) according to step c) is formed.

8. The process according to any of claims 1 to 7, wherein the phase ratio of phase (A) to phase (B) in dispersion (D2) present in stream (S3) is ≤ 0.9 [kg/kg].

9. The process according to any of claims 1 to 7, wherein the phase ratio of phase (A) to phase (B) in dispersion (D2) present in stream (S3) is < 2.5 [kg/kg], stream (S3) being formed in a stirring apparatus by introducing stream (S2) into stream (S1).

10. The process according to any of claims 1 to 9, wherein stream (S1) is obtained from an isomerization, preferably an isomerization in the presence of an ionic liquid, especially an isomerization of methylcyclopentane (MCP) to cyclohexane in the presence of an ionic liquid.

11. The process according to any of claims 1 to 10, wherein stream (S5) obtained according to step e) comprises not more than 10% by weight of phase (A) (based on the amount present in (S3)).

12. The process according to any of claims 1 to 11, wherein stream (S4) obtained from the phase separation unit according to step e) comprises not more than 15% by weight of phase (B) (based on the amount present in (S3)) and/or stream (S4) is recycled into an isomerization.

13. The process according to any of claims 1 to 12, wherein the phase ratio of phase (A) to phase (B) in dispersion (D1) present in stream (S1) is > 3 [kg/kg], preferably > 4 [kg/kg].

14. The process according to any of claims 1 to 13, wherein removal of stream (S2) is followed by passing stream (S5) through a coalescing filter and/or a downstream separator in order to remove residual amounts of phase (A) remaining in stream (S5).

15. The process according to claim 14, wherein a coalescing filter, preferably made from glass fiber or acrylic/phenolic resin, is used, or the downstream separator comprises a knit, preferably a glass fiber knit.

16. The process according to any of claims 1 to 15, wherein cyclohexane is isolated from stream (S5).

## Revendications

1. Procédé de séparation d'une phase (A) d'une phase (B), la phase (A) présentant une viscosité plus élevée que la phase (B), comprenant les étapes suivantes :
a) la préparation d'un courant (S1) contenant une dispersion (D1), dans laquelle la phase (B) est dispersée dans la phase (A), et la phase (B) est contenue à hauteur d'au plus 25 % en poids dans la dispersion,
b) l'introduction d'un courant (S2), contenant au moins 70 % en poids, de préférence au moins 90 % en poids, de phase (B), dans le courant (S1), le courant (S2) étant recyclé depuis l'étape f),
c) pour former un courant (S3), contenant une dispersion (D2), dans laquelle la phase (A) est dispersée dans la phase (B), le rapport de phases entre la phase (A) et la phase (B) dans la dispersion (D2) étant ≤ 3 [kg/kg],
d) l'introduction du courant (S3) dans une unité de séparation de phases,
e) la séparation du courant (S3) dans l'unité de séparation de phases en un courant (S5), contenant au moins 70 % en poids, de préférence au moins 90 % en poids, de phase (B), et en un courant (S4) contenant au moins 70 % en poids, de préférence au moins 90 % en poids, de phase (A),
f) la séparation d'une partie du courant (S5) en tant que courant (S2) et le recyclage du courant (S2) selon l'étape b).

2. Procédé selon la revendication 1, **caractérisé en ce que** la phase (A) présente une viscosité supérieure d'au moins 20 mPas à la phase (B).

3. Procédé selon la revendication 1 ou 2, **caractérisé en ce que** la phase (A) contient au moins un liquide ionique et/ou la phase (B) contient au moins un hydrocarbure.

4. Procédé selon la revendication 3, **caractérisé en ce que** le liquide ionique contenu dans la phase (A) contient en tant que cation un ion ammonium au moins partiellement alkylé ou un cation hétérocyclique et/ou en tant qu'anion un ion chloroaluminate de composition AlₙCl₍₃ₙ₊₁₎ avec 1 < n < 2,5.

5. Procédé selon la revendication 3 ou 4, **caractérisé en ce que** la phase (B) contient en tant qu'hydrocarbure du cyclohexane ou un mélange de cyclohexane avec au moins un autre hydrocarbure choisi parmi le méthylcyclopentane (MCP), le n-hexane, l'iso-hexane, le n-heptane, l'isoheptane ou le diméthylcyclopentane.

6. Procédé selon l'une quelconque des revendications 1 à 5, **caractérisé en ce que** l'unité de séparation de phases est un séparateur de phases.

7. Procédé selon l'une quelconque des revendications 1 à 6, **caractérisé en ce qu'**à l'étape b), l'introduction du courant (S2) dans le courant (S1) a lieu dans un contenant agité ou un mélangeur statique dans lequel le courant (S3) est formé selon l'étape c).

8. Procédé selon l'une quelconque des revendications 1 à 7, **caractérisé en ce que** le rapport de phases entre la phase (A) et la phase (B) dans la dispersion (D2) contenue dans le courant (S3) est ≤ 0,9 [kg/kg].

9. Procédé selon l'une quelconque des revendications 1 à 7, **caractérisé en ce que** le rapport de phases entre la phase (A) et la phase (B) dans la dispersion (D2) contenue dans le courant (S3) est < 2, 5 [kg/kg], le courant (S3) étant formé dans un dispositif agité par introduction du courant (S2) dans le courant (S1).

10. Procédé selon l'une quelconque des revendications 1 à 9, **caractérisé en ce que** le courant (S1) est obtenu à partir d'une isomérisation, de préférence d'une isomérisation en présence d'un liquide ionique, notamment d'une isomérisation de méthylcyclopentane (MCP) en cyclohexane en présence d'un liquide ionique.

11. Procédé selon l'une quelconque des revendications 1 à 10, **caractérisé en ce que** le courant (S5) obtenu selon l'étape e) contient au plus 10 % en poids de phase (A) (par rapport à la quantité contenue dans (S3)).

12. Procédé selon l'une quelconque des revendications 1 à 11, **caractérisé en ce que** le courant (S4) obtenu à partir de l'unité de séparation de phases selon l'étape e) contient au plus 15 % en poids de phase (B) (par rapport à la quantité contenue dans (S3)) et/ou le courant (S4) est recyclé dans une isomérisation.

13. Procédé selon l'une quelconque des revendications 1 à 12, **caractérisé en ce que** le rapport de phases entre la phase (A) et la phase (B) dans la dispersion (D1) contenue dans le courant (S1) est > 3 [kg/kg], de préférence > 4 [kg/kg].

14. Procédé selon l'une quelconque des revendications 1 à 13, **caractérisé en ce qu'**après la séparation du courant (S2), le courant (S5) est conduit dans un filtre à coalescence et/ou un séparateur secondaire afin de séparer les quantités résiduelles restantes de phase (A) dans le courant (S5).

15. Procédé selon la revendication 14, **caractérisé en ce qu'**un filtre à coalescence, de préférence en fibres de verre ou résine d'acrylphénol, est utilisé, ou **en ce que** le séparateur secondaire contient un tricot, de préférence un tricot en fibres de verre.

16. Procédé selon l'une quelconque des revendications 1 à 15, **caractérisé en ce que** du cyclohexane est isolé à partir du courant (S5).
